# EUROPEAN PATENT APPLICATION

(11) **EP 3 225 210 A1**
(43) Date of publication of application: **04.10.2017**
(21) Application number: 17163868.7
(22) Date of filing: 30.03.2017
(51) Int. Cl.: A61F 2/16

(54) **INTRAOCULAR LENS INJECTION INSTRUMENT AND OPHTHALMIC SURGICAL SYSTEM**

(30) Priority: 31.03.2016 JP 2016069881
(71) Applicant: Nidek Co., Ltd., Gamagori-shi, Aichi, 443-0038 (JP)
(72) Inventor: SUZUKI, Nobuo, Gamagori-shi, Aichi 443-0038 (JP)
(74) Representative: Prüfer & Partner mbB Patentanwälte · Rechtsanwälte

(57) **Abstract**

There are provided an intraocular lens injection instrument (200) with improved handleability and an ophthalmic surgical system (1) using the intraocular lens injection instrument. The intraocular lens injection instrument (200) is provided with a casing body (250) having a pressure chamber (RM) variable in its volume varied by a moving wall provided movably inside the casing, a first terminal (254) communicated with the pressure chamber (RM) to connect the pressure chamber with an aspiration pump (40) via a tube (7), and a movable member (294) having one end connected with a moving wall (291) and the other end to directly or indirectly push out an intraocular lens (400), the intraocular lens injection instrument (200) being configured such that the moving wall (291) is movable by a negative pressure in the pressure chamber (RM) to be generated by an aspiration force of the aspiration pump (40) to convert the aspiration force of the aspiration pump (40) into a linear movement of the movable member (294).

## Description

### BACKGROUND

### Technical Field

The present disclosure relates to an intraocular lens injection instrument to inject an intraocular lens (IOL) into an eye and an ophthalmic surgical system using the intraocular lens injection instrument.

### Related Art

There has been known an intraocular lens injection instrument to inject an IOL into a patient's eye. For example, Patent Document 1 provides an intraocular lens injection instrument provided with an operating-pressure control member. Specifically, the number of washers used for flattening or deforming an O ring is adjusted so that an operating power of a push-out mechanism is controlled.

### Related Art Documents

### Patent Documents

Patent Document 1: JP 2004-113610 A

### SUMMARY

However, the IOL injection instrument of Patent Document 1 requires a force of an operator's hand to push out the IOL. Namely, a push-out speed to push the IOL depends on the force of the operator's hand pushing a push-out part, for example. For an operator who is unfamiliar with handling the IOL injection instrument, accordingly, it is difficult to control a strength to push the push-out part, making it further difficult to feed the IOL through the IOL injection instrument at a stable push-out speed.

The present disclosure has been made in view of the above-mentioned problem of the prior art and to provide an intraocular lens injection instrument with an improved handleability and an ophthalmic surgical system.

To address the above problem, the present disclosure has the following configurations.
(1) An intraocular lens injection instrument comprising: a casing body including a pressure chamber which is variable in its volume by movement of a moving wall provided movably in a housing; a first terminal configured to communicate with the pressure chamber for connecting an aspiration pump via a tube; and a movable member having one end connected with the moving wall and the other end configured to directly or indirectly push an intraocular lens, wherein the moving wall is movable by a negative pressure to be generated by an aspiration force of the aspiration pump, and the moving wall is configured to be moved to convert the aspiration force of the aspiration pump into linear movement of the movable member. Further developments of the present disclosure are given in the dependent claims.

According to the present disclosure, an intraocular lens injection instrument with the improved handleability and an ophthalmic surgical system using the IOL injection instrument are provided.

### BRIEF DESCRIPTION OF THE DRAWINGS

FIG. 1 is an external perspective view of an ophthalmic surgical system in the present embodiment;
FIG. 2 is a schematic configurational view of the ophthalmic surgical system in FIG. 1;
FIG. 3 is an external perspective view of a second handpiece;
FIG. 4 is a schematic sectional view of an injector part;
FIG. 5 is a schematic sectional view of a casing body of the second handpiece;
FIG. 6 is a schematic explanatory view of the ophthalmic surgical system using the second handpiece;
FIG. 7 is a flow chart of a method for using the ophthalmic surgical system in FIG. 1;
FIG. 8 is a flow chart showing the control carried out by a controller;
FIG. 9 is a graph related to an output signal from a pressure sensor;
FIG. 10 is a flow chart showing the control carried out by the controller;
FIG. 11 is a graph related to the output signal from the pressure sensor;
FIG. 12 is a schematic view for explaining a relation of an aspiration pump and a plunger;
FIG. 13A is a schematic perspective view of a second handpiece in a modified example; and
FIG. 13B is a sectional view taken along a line B-B in FIG. 13A.

### DETAILED DESCRIPTION OF THE EXEMPLARY EMBODIMENTS

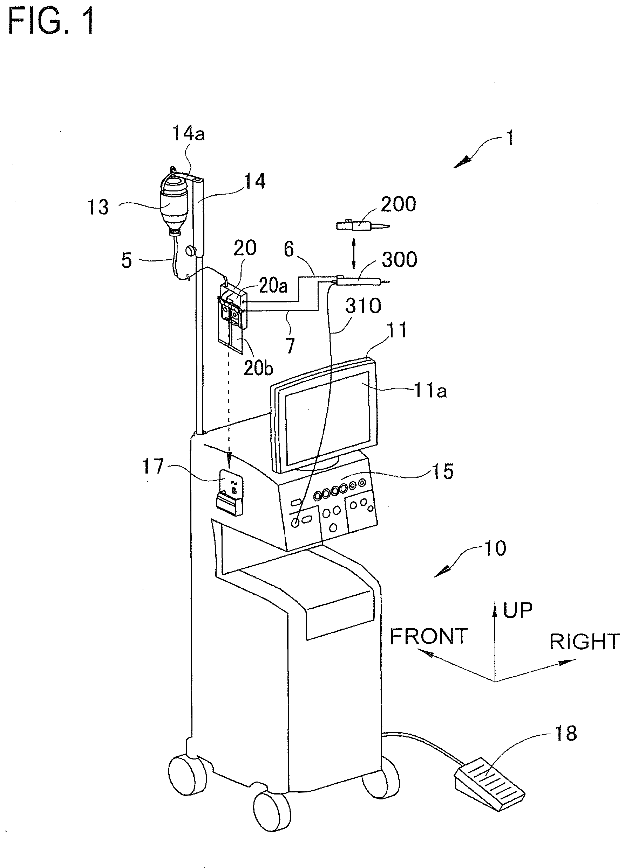

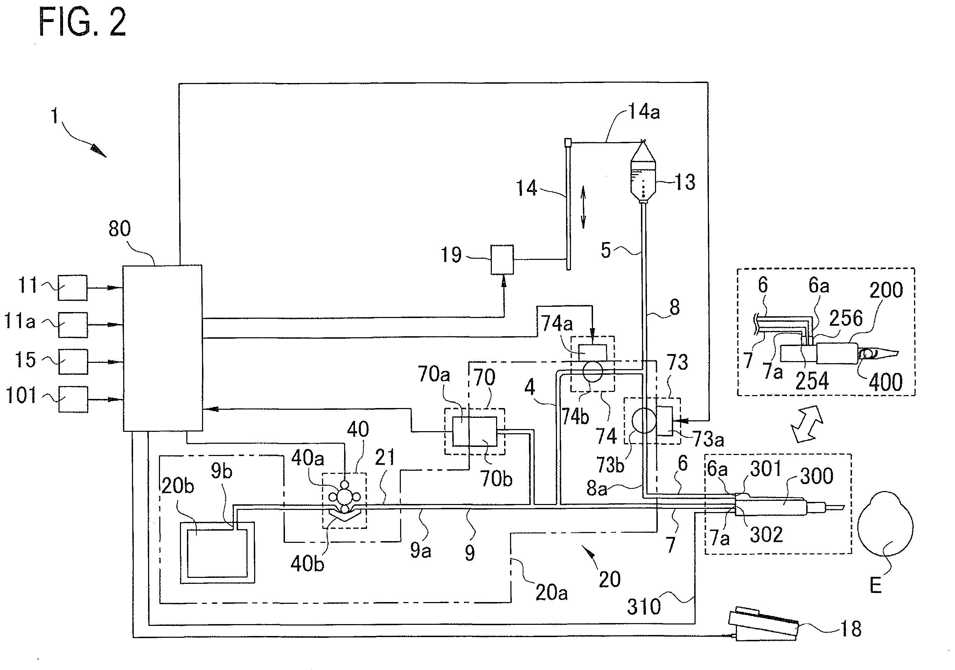

A typical embodiment according to the present disclosure is explained below with reference to the accompanying drawings. FIG. 1 is an external perspective view of an ophthalmic surgical system 1 of the present embodiment when it is seen from obliquely upward left. FIG. 2 is a schematic configurational view of the ophthalmic surgical system 1 shown in FIG. 1.

### <Ophthalmic surgical system>

The ophthalmic surgical system 1 of the present embodiment includes a cataract surgical apparatus body 10, a cassette 20, and a second handpiece 200. The ophthalmic surgical system 1 of the present embodiment further includes a supply bottle 13, a foot pedal 18, and a first handpiece 300. The system 1 of the present embodiment is connectable with at least any one of the first handpiece 300 and the second handpiece 200. The first handpiece 300 of the present embodiment is, for example, used for aspirating and removing a nucleus of lens from a patient's eye E. The second handpiece 200 (an intraocular lens injection instrument) of the present embodiment is, for example, used for injecting an intraocular lens (IOL) 400. The cassette 20 can be shared for both the first handpiece 300 and the second handpiece 200 in the system 1 of the present embodiment. The cassette 20 of the present embodiment includes at least a part of an aspiration passage 9 which is used for aspirating a fluid from the first handpiece 300 or the second handpiece 200. The cassette 20 of the present embodiment is disposable, and thus time and labor for cleaning or sterilizing after surgery is restrained.

### <Cataract surgical apparatus body>

The cataract surgical apparatus body 10 of the present embodiment is provided with an aspiration pump 40 (see FIG. 2) configured to aspirate a fluid from the first handpiece 300 or the second handpiece 200. The cataract surgical apparatus body 10 of the present embodiment is further provided with a monitor 11, a touch screen 11 a, a connection panel 15, a holding unit 17, a pole 14, an arm 14a, the foot pedal 18, and a controller 80 (see FIG. 2). The monitor 11 of the present embodiment displays a screen showing setting surgical conditions, a list of surgical results (results of driving the surgical apparatus), and others. The touch screen 11 a of the present embodiment is input with various operations input by an operator. The connection panel 15 of the present embodiment is provided with a plurality of connectors. The connection panel 15 is connectable with, for example, a cable 310 or the like to control the first handpiece 300. The holding unit 17 of the present embodiment is configured to be mounted with the cassette 20.

The holding unit 17 of the present embodiment includes a pressing mechanism 73a, a pressing mechanism 74a, a deformation detection part 70a, a rotation part 40a, and a tube receiver 40b (see FIG. 2). The pole 14 of the present embodiment supports the supply bottle 13. The supply bottle 13 of the present embodiment contains a fluid. To be specific, the supply bottle 13 of the present embodiment contains a supply liquid. In the present embodiment, a physiological saline solution is used as the supply liquid. The arm 14a of the present embodiment is configured to hang the supply bottle 13 on an upper end of the pole 14. The foot pedal 18 is used for an operator's adjustment of an aspiration flow rate of the supply liquid, for example. Injection of the IOL 400, which has been placed in the second handpiece 200, may be started by an operator's stepping of the foot pedal 18. An injection speed of the IOL 400 may be controlled by a pressed amount of the foot pedal 18. The foot pedal 18 may be used for performing ultrasonic vibration or aspiration movement of a movable tip provided on a front end of the first handpiece 300, for example.

### <Cassette>

The cassette 20 of the present embodiment includes a cassette body 20a, a bag 20b, a tube 5, a tube 6, and a tube 7 (see FIG. 2). The cassette body 20a of the present embodiment includes an elastic member 73b, an elastic member 74b, a pressure deformation part 70b, and a tube 21. The cassette body 20a includes a passage 8a which is at least a part of the supply passage 8, a passage 9a which is at least a part of the aspiration passage 9, and a pinch passage 4, the passages 8a, 9a, and 4 being formed of tubes or the like. The elastic member 73b is a part of the supply passage 8, and the elastic member 74b is a part of the aspiration passage 9. Each of the tube 5, the tube 6, the tube 7, the tube 21, the passage 8a, and the passage 9a has flexibility and bendability. In the present embodiment, the tube 5, the tube 6, the tube 7, the tube 21, the passage 8a, and the passage 9a are made of silicone tube. The cassette 20 of the present embodiment is configured with components made of resin as a material. The cassette 20 of the present embodiment may be called as a disposable unit.

### <Controller>

The controller 80 of the present embodiment is explained. The controller 80 of the present embodiment is in charge of operating the entire ophthalmic surgical system 1. The controller 80 of the present embodiment is connected with the monitor 11, the touch screen 11a, the connection panel 15, the foot pedal 18, a drive mechanism 19, a memory 101, the pressing mechanism 73a, the pressing mechanism 74a, the deformation detection part 70a, and the rotation part 40a. The controller 80 of the present embodiment may be connected with the first handpiece 300, and in connection, the controller 80 and the first handpiece 300 are connected by the cable 310. The memory 101 of the present embodiment is a storage member (a storage part). The memory 10 may store a program for performing surgery and various surgical information obtained through surgical operation of the cataract surgical apparatus body 10, for instance. The controller 80 of the present embodiment has a function as a display control member to control contents to be displayed on the monitor 11. Specifically, the controller 80 may change the display contents displayed on the monitor 11 based on an input signal from the touch screen 11 a and others.

The controller 80 of the present embodiment reads out a program stored in the memory 101 and functions as a processor to control the rotation part 40a and others. As the processor, a CPU (a microprocessor), a DSP (a digital signal processor), a PLD (a programmable logic device), and others may be adapted. Alternatively, a processor may be a combination of the CPU, the DSP, the PLD, and others. The processor (the controller 80) may have a storage member to store the program. The controller 80 of the present embodiment also functions as a drive control member to control driving of the aspiration pump 40 based on an output signal from the pressure sensor 70. As one example, the controller 80 of the present embodiment is configured to attenuate an aspiration force generated by the aspiration pump 40 when the pressure detected by the pressure sensor 70 exceeds a predetermined value.

### <First handpiece>

In the present embodiment, as the first handpiece 300, a US handpiece (an ultrasonic wave handpiece) is used. The US handpiece of the present embodiment is configured to emulsify, aspirate, and then remove the nucleus of lens, which has become opaque and hardened due to cataract, by ultrasonic wave vibration of a cracking tip provided on a front end of the handpiece. In other words, the first handpiece 300 may be used for the cataract surgery (see JP 2000-237228 A as one example). As an alternative, an I/A handpiece (a handpiece for perfusion-aspiration) may be adapted, for example (see JP 06(1994)-225903 A as one example). The first handpiece 300 of the present embodiment is connected with the tube 6, the tube 7, and the cable 310. More specifically, a terminal 301 of the first handpiece 300 is connected with an end portion 6a (a downstream end) of the tube 6, and a terminal 302 of the first handpiece 300 is connected with an end portion 7a (an upstream end) of the tube 7. The first handpiece 300 includes an outflow hole provided on its front end and this outflow hole is communicated with the terminal 301. The first handpiece 300 further includes an inflow hole provided on its front end and this inflow hole is communicated with the terminal 302. The outflow hole is, for example, configured to supply a fluid (a supply liquid) into a patient's eye E. The inflow hole is, for example, configured to aspirate a fluid (a waste liquid) from the patient's eye E.

### <Supply passage and Supply unit>

The ophthalmic surgical system 1 of the present embodiment is provided with a supply unit to supply a fluid to at least any one of the first handpiece 300 and the second handpiece 200. The supply unit of the present embodiment includes the drive mechanism 19, the pole 14, the arm 14a, the supply bottle 13, the tube 5, the passage 8a in the cassette body 20a, a supply valve 73, and the tube 6 (see FIG. 2). In the present embodiment, the supply passage 8, in which the fluid supplied by the supply unit flows, is formed of the tube 5, the passage 8a in the cassette body 20a, and the tube 6. In the present embodiment, the fluid (the supply liquid) flows in the supply passage 8. For instance, the supply liquid flows from the supply bottle 13 (on an upstream side) to the handpiece (the first handpiece 300 or the second handpiece 200 on a downstream side).

In the present embodiment, driving the drive mechanism 19 by the controller 80 causes up and down movement of the pole 14. Owing to this up and down movement of the pole 14, the supply pressure of the supply liquid flowing from the supply bottle 13 is controlled. Namely, the ophthalmic surgical system 1 of the present embodiment has a supply-pressure control member to control the supply pressure of the fluid (the supply liquid). The supply pressure may be controlled by use of a pressure source (such as a gas source) applying pressure to the supply liquid. In the present embodiment, the supply valve 73 is provided on the way of the supply passage 8. The supply valve 73 of the present embodiment is configured to regulate the flow rate of the fluid flowing from the end portion 6a of the tube 6. Namely, the supply valve 73 of the present embodiment is configured to control the supply pressure of the supply passage 8 (on the downstream side of the supply valve 73). In the present embodiment, adjustment performed by the supply valve 73 includes shut-off of the valve. The supply valve 73 of the present embodiment includes the pressing mechanism 73a and the elastic member 73b. In the present embodiment, when the pressing mechanism 73a presses the elastic member 73b, the elastic member 73b is deformed to reduce its sectional area (a section orthogonally intersecting an upstream and downstream direction) of the supply passage 8. Thus, the flow rate of the fluid flowing through the supply passage 8 is controlled by the supply valve 73.

### <Aspiration passage and Aspiration member>

The ophthalmic surgical system 1 of the present embodiment is provided with an aspiration member to aspirate the fluid from at least any one of the first handpiece 300 and the second handpiece 200. The aspiration member of the present embodiment is configured to generate an aspiration pressure of at most 600 mmHg. Further, the aspiration member of the present embodiment is configured to aspirate the fluid at a flow rate of at most 60 mil/min. The aspiration member of the present embodiment includes the aspiration pump 40. The aspiration pump 40 of the present embodiment is configured to set the aspiration pressure in a range of 0 to 600 mmHg by the control of the controller 80. The aspiration pump 40 of the present embodiment is further configured to set an aspiration flow rate in a range of 0 to 60 ml/min by the control of the controller 80.

The aspiration member of the present embodiment includes the tube 7, the passage 9a in the cassette 20, the tube 21, the rotation part 40a, the tube receiver 40b, and the bag 20b (see FIG. 2). In the present embodiment, the aspiration passage 9, in which the fluid aspirated by the aspiration member flows, is formed of the tube 7, the passage 9a in the cassette body 20a, and the tube 21. In the present embodiment, there is a case that the fluid (gas or liquid) supplied by the supply unit is included in the fluid flowing in the aspiration passage 9. For example, when the first handpiece 300 is used, there is a case that an organ of the patient's eye E is included in the fluid flowing in the aspiration passage 9.

In the present embodiment, the aspiration pump 40 is provided on the way of the aspiration passage 9. To be specific, the aspiration pump 40 of the present embodiment includes the rotation part 40a, the tube receiver 40b, and the tube 21. The aspiration pump 40 of the present embodiment is sometimes called as a peristaltic aspiration pump, a peristaltic pump, or the like.

The aspiration pump 40 of the present embodiment is explained. The rotation part 40a of the present embodiment is provided with a rotation table, drawing members, a rotary shaft, and a motor. The rotation table of the present embodiment is provided with the four drawing members. The rotation table is provided on its center with the rotary shaft. The rotary shaft is connected to the motor (such as a stepper motor). The motor is connected to the controller 80. The four drawing members of the present embodiment are placed on the same circumference of the rotation table. The four drawing members of the present embodiment are each placed in every 90-degree position about the rotary shaft.

The aspiration pump 40 of the present embodiment is configured such that the tube 21 is trained or placed on the tube receiver 40b so that the tube 21 is held between the tube receiver 40b and the drawing member included in the rotation part 40a, thus drawing or pulling by the drawing member and aspirating the tube 21. In other words, the fluid inside the tube is moved by the peristaltic movement of the tube (the passage). In the present embodiment, when the motor of the rotation part 40a is driven by the controller 80, the rotation table is rotated to bring the drawing members to successively flatten the tube 21. The drawing members of the present embodiment make rotary movement to clog or deform the aspiration passage 9. Thus, the fluid inside the tube 21 is transmitted.

As mentioned above, the aspiration pump 40 generates the aspiration pressure to aspirate the fluid from the first handpiece 300 or the second handpiece 200 in the present embodiment. The rotation table of the aspiration pump 40 of the present embodiment is allowed to be rotated in a reverse direction. This reverse rotation makes it possible to utilize the aspiration pump 40 of the present embodiment as a discharge pump. Accordingly, the aspiration pump 40 of the present embodiment may be called as a pump. For example, utilizing the aspiration pump 40 (pump) of the present embodiment as a discharge pump contributes to relaxing the aspiration pressure (the negative pressure) in the aspiration passage 9.

In the present embodiment, the pressure sensor 70 is provided on the way of the aspiration passage 9. Specifically, the pressure sensor 70 of the present embodiment is provided on the way of the aspiration passage between the end portion 7a and the aspiration pump 40. The pressure sensor 70 of the present embodiment includes the deformation detection part 70a and the pressure deformation part 70b. The pressure sensor 70 of the present embodiment is used for the controller 80 to detect pressure in the aspiration passage 9. The pressure deformation part 70b includes a deformation part which is to be deformed at a deformation amount corresponding to the pressure in the aspiration passage 9. The deformation detection part 70a detects the deformed amount of the deformation part. In the present embodiment, an end portion 9b (a downstream end) of the aspiration passage 9 is connected with the bag 20b. The bag 20b of the present embodiment is gathered with the fluid aspirated by the aspiration member. The bag 20b of the present embodiment is made of transparent and bendable resin (vinyl). The bag 20b of the present embodiment is hung on a casing (on an outer face) of the cassette body 20a (see FIG. 1).

### <Pinch passage>

A pinch passage 4 of the present embodiment connects the supply passage 8 with the aspiration passage 9. The pinch passage 4 of the present embodiment is used for relaxing the pressure (the negative pressure) of the aspiration passage 9. In the present embodiment, a pinch valve 74 is provided on the way of the pinch passage 4. The pinch valve 74 of the present embodiment includes the pressing mechanism 74a and the elastic member 74b. The pinch valve 74 of the present embodiment is configured to control a flow rate of the fluid flowing through the pinch passage 4. In the present embodiment, the pinch valve 74 is formed from the same parts or components with the supply valve 73, and thus detailed explanation thereof is omitted. The ophthalmic surgical system 1 may dispense with the pinch passage 4.

### <Second handpiece>

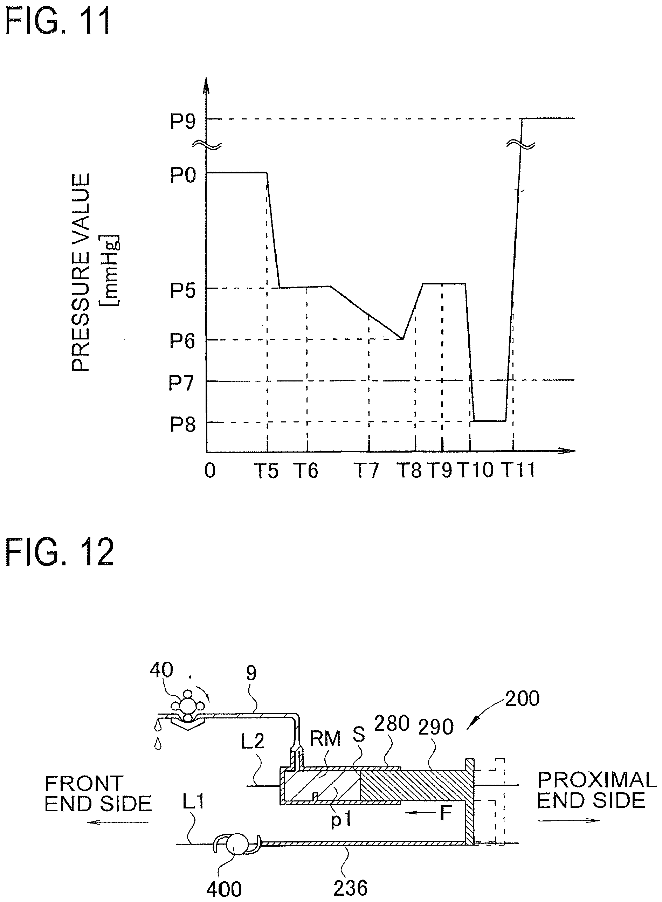

Firstly, a relation of the aspiration pump 40 and a plunger 236 of the present embodiment is simply explained with reference to FIG. 12 (a schematic drawing for explanation). A piston part 290 is movable along a moving axis L2 in a casing of a cylinder part 280. A pressure chamber RM defined by the piston part 290 and the cylinder part 280 is connected to the aspiration pump 40 via the aspiration passage 9. The aspiration passage 9 and the pressure chamber RM are filled with a liquid. The piston part 290 is connected with the plunger 236. The cylinder part 280 (a fixed member) is a cylindrical component one end (a front end) of which is closed, and the piston part 290 (a moving member) is a tubular component. It is noted that movement of the fluid is restricted in a clearance between the piston part 290 and the cylinder part 280.

When the aspiration pump 40 generates a pressure p1 (a negative pressure), a pressure value in the aspiration passage 9 and the pressure chamber RM becomes the pressure p1. The piston part 290 is applied with a force F corresponding to an area S (an area of a front end face of the piston part 290) and the pressure p1. The force F is applied to a front end of the moving axis L2. The force F is exerted to move the piston part 290 toward the front end of the moving axis L2 and move the plunger 236 toward a front end of the moving axis L1. The moving axis L1 and the moving axis L2 are in parallel to each other. The plunger 236 is configured to push the IOL 400 placed on the moving axis L1 toward the front end of the moving axis L1 with the force F. Thus, the plunger 236 is moved by the aspiration force of the aspiration pump 40. When the aspiration pump 40 performs aspiration at an aspiration flow rate Q, the piston part 290 and the plunger 236 move at a moving speed SPD (SPD=Q/S). When it is assumed that the pressure p1 is a fixed value, the larger the area S is, the larger the push-out force (the force F) of the plunger 236 becomes. When it is assumed that the aspiration flow rate Q is a fixed value, the larger the area S is, the slower the moving speed (the moving speed SPD) of the plunger 236 becomes.

In one example, when the aspiration flow rate Q is 60 (ml/min) (i.e., 1000 mL/second) and an inner diameter of the cylinder part 280 is 15 mm, which means the area S is 176.6 mm², the moving speed SPD of the plunger 236 is 5.66 (mm/sec) (SPD=1000/176.6). Further, when the condition of the aspiration flow rate Q is same as above and the inner diameter of the cylinder part 280 is 12 mm, which means the area S is 113.0 mm², the moving speed SPD of the plunger 236 is 8.85 (mm/sec) (SPD=1000/113).

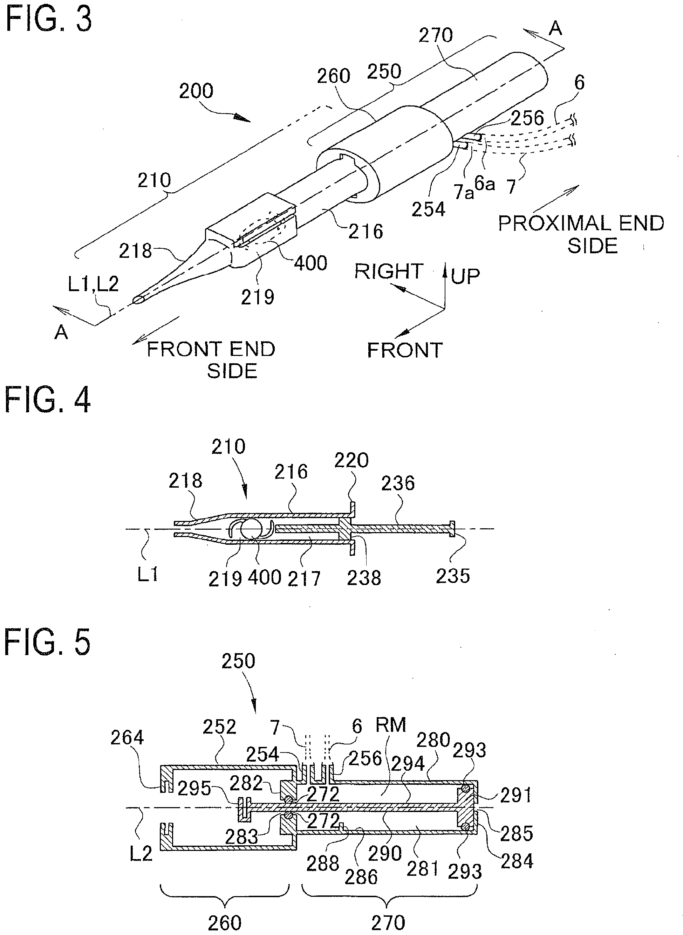

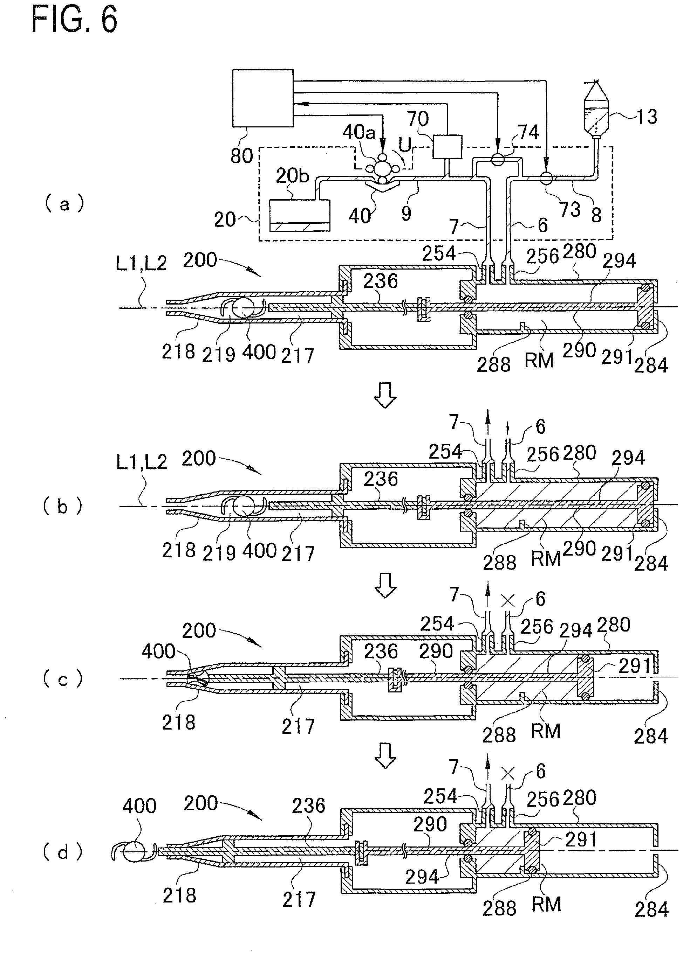

The second handpiece 200 of the present embodiment is explained with reference to FIGs. 3 to 6. FIG. 3 is an external perspective view of the second handpiece 200 when it is seen from obliquely upward left. FIG. 4 is a sectional view of the injector part 210, corresponding to a front-end-side cross section taken along a line A-A in FIG. 3. FIG. 5 is a sectional view of the casing body 250, corresponding to a proximal-end-side cross section taken along a line A-A in FIG. 3. FIG. 6 includes sectional views of the second handpiece 200 in (a) to (d), each corresponding to a cross section taken along a line A-A in FIG. 3. FIGs. 5 and 6 are schematic sectional views in which only each position of the first terminal 254 and the second terminal 256 is moved in a circumferential direction about the moving axis L2.

The second handpiece 200 of the present embodiment is used for injecting the IOL 400 into the patient's eye E. Specifically, the second handpiece 200 of the present embodiment is used for injecting the IOL 400 which has been stored inside the second handpiece 200 into the patient's eye E. In the present embodiment, the IOL 400 is injected into the patient's eye E by the aspiration force of the aspiration member which is connected to the second handpiece 200. To be more specific, the second handpiece 200 of the present embodiment brings the IOL 400 to move toward the front end of the moving axis L1 by use of the negative pressure generated by the aspiration pump 40 (see FIG.2), and thus the IOL 400 can be injected (discharged) from the second handpiece 200.

The second handpiece 200 of the present embodiment includes the injector part 210 and the casing body 250 (see FIG. 3). In the present embodiment, the casing body 250 is attached to a proximal end of the injector part 210. Herein, the injector part 210 of the present embodiment can inject the IOL 400 into the patient's eye E by itself (only by the injector part 210). However, in that case, a force of an operator's hand (fingers) is to be used to move the IOL 400 into the patient's eye E from the injector part 210. In other words, for injection of the IOL 400, the operator may choose any one configuration of injecting the IOL 400 only by use of the injector part 210 (pressing the IOL 400 only by the force of the operator's hand) or using the ophthalmic surgical system 1 (pressing the IOL 400 by the force of other than the operator's hand).

Attachment of the injector part 210 and the casing body 250 to form the second handpiece 200 of the present embodiment is performed during the manufacturing process of the second handpiece 200. Alternatively, the injector part 210 and the casing body 250 may be attached at the site of use. As another alternative, another type of injector part other than the injector part 210 may be attached. For example, the injector part may be a cartridge type (configured only with an injection part 218 and a setting part 219) (see JP 2004-113610 A as one example). The second handpiece 200 of the present embodiment is made of resin, and thus disposal of the second handpiece 200 is made easily. The second handpiece 200 may however include metal or the like in its part of material. The second handpiece 200 of the present embodiment has been loaded with the IOL 400 in advance, but alternatively, loading of the IOL 400 may be performed at the site of use.

The IOL 400 of the present embodiment is explained. The IOL 400 of the present embodiment includes an optical part and support parts. The optical part provides optical characteristics to the patient's eye E. The support parts support the optical part in the patient's eye E. The optical part of the IOL 400 of the present embodiment is of a disk-like shape, and the support part is of a loop-like shape. The support parts are connected to a circumference of the optical part. The IOL 400 of the present embodiment is formed integrally by the optical part and a pair of support parts (which is sometimes called as a one-piece type). The IOL 400 of the present embodiment has flexibility. As a material for this type of IOL 400, there is a polymerized material including acrylate ester and methacrylate ester or the like as principal ingredients, for example. Types of the IOL 400 are not limited to this. For example, an optical part and a support part may be made of different materials, for example (which is sometimes called as a three-piece type).

### <Injector part>

The injector part 210 of the present embodiment is explained with reference to FIGs. 3 and 4. The IOL 400 of the present embodiment has been loaded when manufacturing the injector part 210 (the second handpiece 200). Namely, the IOL 400 has been preset (pre-loaded) in the injector part 210. Thus, a user's labor of loading the IOL 400 in the injector part 210 is restrained. The injector part 210 of the present embodiment includes a barrel body 216 and the plunger 236. The barrel body 216 of the present embodiment is a cylindrical component, and a hollow part 217 communicates through a proximal end to a front end of the barrel body 216 (see FIG. 4). The plunger 236 of the present embodiment is a bar-like component, and its front end portion enters in the hollow part 217. In the present embodiment, the plunger 236 is configured to move along the moving axis L1 inside the barrel body 216 (in the hollow part 217), so that the IOL 400 set in the hollow part 217 is pushed out toward the front end and injected from the front end of the barrel body 216.

In the present embodiment, a setting part 219 is provided on the way of the hollow part 217 extending along the moving axis L1. In the present embodiment, the IOL 400 before injection is placed (set) on the setting part 219. A width of the setting part 219 (a width in a left and right direction in FIG. 3) is made larger than a width of the IOL 400 in a placed position on the setting part 219. There is provided an injection part 218 on a front end side of the barrel body 216. The IOL 400 is to be injected from atip end of the injection part 218 (the front end of the hollow part 217). The injection part 218 of the present embodiment is shaped tapered toward its tip end. Accordingly, a sectional area of the hollow part 217 (a surface orthogonal to the moving axis L1) is also shaped tapered toward the front end. In the present embodiment, this inner hollow shape of the injection part 218 leads to deformation of the IOL 400 to make its sectional area (a surface orthogonal to the moving axis L1) smaller, and then the IOL 400 is injected from the tip end of the injection part 218. On a proximal end of the barrel body 216, a holding part 220 extending away from the moving axis L1 is provided. When injection of the IOL 400 is to be carried out only by the injector part 210, the holding part 220 is used for the operator to hold the injector part 210. On the other hand, when the injection of the IOL 400 is to be carried out by the combination of the injector part 210 and the casing body 250, the holding part 220 is used for attachment of the casing body 250 to the injector part 210.

The plunger 236 of the present embodiment is used for pushing out the IOL 400. The plunger 236 of the present embodiment is a bar-like component. The plunger 236 is placed on the moving axis L1. The plunger 236 is provided with a guide part 238 extending away from the moving axis L1 in a position closer to the proximal end side than the front end of the plunger 236. A sectional area (a cross section orthogonal to the moving axis L1) of the guide part 238 is made slightly smaller than a sectional area of the hollow part 217. The guide part 238 is, for example, configured to restrain wobbling of the plunger 236 in a direction away from the moving axis L1. On a proximal end of the plunger 236, a pressing part 235 extending in a direction away from the moving axis L1 is provided. When the injection of the IOL 400 is to be performed only by the injector part 210, operator's fingers are to be held against the pressing part 235. On the other hand, when the injection of the IOL 400 is to be performed by the combination of the injector part 210 and the casing body 250, the pressing part 235 is used for attachment of the casing body 250 to the injector part 210.

### <Casing body>

The casing body 250 of the present embodiment is explained with reference to FIGs. 3 and 5. The casing body 250 of the present embodiment includes a connection part 260 and a driving-force conversion part 270. The connection part 260 of the present embodiment is configured to join the injector part 210 to the driving-force conversion part 270. The driving-force conversion part 270 (a conversion mechanism) of the present embodiment is configured to convert the aspiration force generated by the aspiration pump 40 (the aspiration member) into linear movement of the plunger 236 (moving in a direction along the moving axis L1). Thus, the IOL 400 is moved by the aspiration force generated in the aspiration pump 40. To be more specific, the aspiration pump 40 of the present embodiment is configured to change the pressure inside the pressure chamber RM which is defined by an inner wall 286 (a fixed wall) of the cylinder part 280 and a moving wall 291 of the piston part 290. The casing body 250 of the present embodiment includes the pressure chamber RM which is changeable in its capacity (volume) by the moving wall 291 provided movably inside the casing. Changes in the pressure make the moving wall 291 linearly move along the moving axis L2.

The moving wall 291 and the movable member 294 of the present embodiment are integrally formed, and thus the movable member 294 and the moving wall 291 are configured to move in the same direction. To a front end of the movable member 294, the plunger 236 is to be connected. When the moving wall 291 moves along the moving axis L2, the plunger 236 moves along an axis (the moving axis L1) parallel to the moving axis L2. The moving axis L1 and the moving axis L2 are coaxial to each other in the present embodiment, but alternatively, the moving axis L1 and the moving axis L2 may not be in parallel. As one alternative example, the casing body 250 may include a direction conversion mechanism (such as a seesaw mechanism). This direction conversion mechanism may move the plunger 236 in a front direction in association with the movement of the piston part 290 in a downward direction (see FIG. 3 for the direction). As another alternative example, the direction conversion mechanism may move the plunger 236 in the front direction in association with the movement of the piston part 290 in a backward direction. When the casing body 250 includes the direction conversion mechanism, an entire length (in a front and rear direction in FIG. 3) of the second handpiece 200 can be made short.

The driving-force conversion part 270 of the present embodiment includes the cylinder part 280 and the piston part 290. The cylinder part 280 of the present embodiment is a tubular shape, and the cylinder part 280 is provided its inside with a hollow part 281 which is surrounded by the inner wall 286 of the cylinder part 280. The hollow part 281 is a columnar shape. A sectional area (a cross section orthogonal to the moving axis L2) of the hollow part 281 is preferably 1 cm² or more and 2cm² or less. Small sectional area of the hollow part 281 makes it easy for the operator to handle the second handpiece 200, for example. In the present embodiment, the sectional area (a cross section orthogonal to the moving axis L2) of the hollow part 281 is 1.8 cm², and the cross section is in an almost circular shape. The piston part 290 is allowed to move along the moving axis L2 in the hollow part 281. In the present embodiment, a front wall 282 is formed on a front end of the hollow part 281. The front wall 282 is a part of the inner wall 286. The front wall 282 is formed with a hole 283. The hole 283 is formed in a central portion of the front wall 282. The hole 283 intersects the moving axis L2, and the movable member 294 passes through the hole 283. A rear wall 284 is formed on a proximal end of the hollow part 281. The rear wall 284 is a part of the inner wall 286. The rear wall 284 is formed with a hole 285. The hole 285 allows gas to flow in the hollow part 281 from outside of the cylinder part 280, for example. Further, the rear wall 284 functions as a moving limit of the piston part 290 (on a proximal end side of the moving axis L2).

In the present embodiment, a restriction member 288 is provided between the front wall 282 and the rear wall 284. The restriction member 288 protrudes from the inner wall 286 toward the hollow part 281 (toward the moving axis L2). In the present embodiment, the moving wall 291 having kept moving toward the front end of the moving axis L2 is made to be in contact with the restriction member 288. Namely, the restriction member 288 functions as a moving limit of the piston part 290 (on the front end side of the moving axis L2). In other words, the restriction member 288 of the present embodiment is provided for the purpose of defining the moving limit in the linear movement of the moving wall 291. The first terminal 254 and the second terminal 256 are provided between the front wall 282 and the restriction member 288. The first terminal 254 is connected with the tube 7 for aspirating the fluid, and the second terminal 256 is connected with the tube 6 for supplying the fluid. Each of the first terminal 254 and the second terminal 256 is formed with a through hole to communicate with the hollow part 281 (the pressure chamber RM). The first terminal 254 communicates with the pressure chamber RM in its front part located far front than a moving limit position of the moving wall 291. The first terminal 254 may be alternatively provided in the piston part 290. Specifically, another configuration may be provided such that a front surface of the moving wall 291 (a wall surface of the moving wall 291 facing the front end of the moving axis L2, i.e., a wall surface of the moving wall defining the pressure chamber RM) is formed with a through hole, and the first terminal 254 (connected with the tube 7 for aspirating the fluid) which is provided on a rear surface of the moving wall 291 (a wall surface facing the proximal end of the moving axis L2, i.e., the wall surface of the moving wall 291 not defining the pressure chamber RM) may be communicated with the through hole. In other words, during movement of the piston part 290, the aspiration passage 9 only has to be hardly closed partly or entirely. Herein, when the inner wall 286 is in a conical shape tapered toward the first terminal 254, gas (air) inside the pressure chamber RM is easily discharged to the aspiration passage 9.

The piston part 290 of the present embodiment is a bar-like shape. The piston part 290 of the present embodiment is placed on the moving axis L2. In the present embodiment, the piston part 290 is provided on its proximal end with the moving wall 291 of a disk-like shape. On a front end side of the moving wall 291, the columnar-shaped movable member 294 is connected. The movable member 294 extends along the moving axis L2 toward its front end. Irrespective of the moving position of the moving wall 291, the front end portion of the movable member 294 comes out of the front wall 282. A fitting portion 295 is formed on the front end portion of the movable member 294 which has come out of the front wall 282. The fitting portion 295 is configured to be fitted with the pressing part 235 (see FIG. 4). Specifically, the movable member 294 and the plunger 236 are connected via the fitting portion 295. The movable member 294 of the present embodiment is connected its one end with the moving wall 291. The other end of the movable member 294 is used for directly or indirectly pushing the IOL 400. Namely, the movable member 294 of the present embodiment pushes the IOL 400 in an indirect manner. The movable member 294 of the present embodiment pushes (injects) the IOL 400 via the plunger 236. The shape of the fitting portion 295 corresponds to the shape of the pressing part 235. For example, the fitting portion 295 may be detachable from the movable member 294. There may be a several types of the fitting portions 295. A fitting portion corresponding to a configuration of the injector part 210 may be selectively attached to the movable member 294 when manufacturing the second handpiece 200 or at the site of use.

In the present embodiment, an O ring 293 (a sealing member) is placed between the inner wall 286 and the moving wall 291. The O ring 293 of the present embodiment fills a clearance between the moving wall 291 and the cylinder part 280. Specifically, the O ring 293 restricts the fluid from flowing through the clearance between the moving wall 291 and the cylinder part 280. In the present embodiment, an Oring 272 (a sealing member) is placed between the movable member 294 and the front wall 282. The Oring 272 of the present embodiment fills a clearance between the movable member 294 and the front wall 282. Specifically, the Oring 272 restricts the fluid from flowing through the clearance between the movable member 294 and the front wall 282. In the present embodiment, at least a part of region (space) in the hollow part 281 surrounded by the inner wall 286, the piston part 290, and the O rings (the O ring 272 and the O ring 293) is defined as the pressure chamber RM. The movable member 294 of the present embodiment penetrates through the pressure chamber RM. The ophthalmic surgical system 1 of the present embodiment is configured such that the piston part 290 is moved toward the front end of the moving axis L2 by reducing the pressure in the pressure chamber RM so that the IOL 400 is moved (injected) toward the front end of the moving axis L1.

The connection part 260 of the present embodiment includes a tube part 252 and a fitting portion 264. In the present embodiment, the cylindrical-shaped tube part 252 is connected to the front end side of the cylinder part 280, and the tube part 252 extends toward the front end of the moving axis L2. A front end of the tube part 252 is provided with the plate-like fitting portion 264 bent toward the moving axis L2. A part of the fitting portion 264 facing the moving axis L2 is in a recessed or uneven shape. The recessed shape of the fitting portion 264 is formed to be fitted with the holding part 220 to couple a barrel body 216 with the tube part 252. The recessed shape of the fitting portion 264 of the present embodiment corresponds to the shape of the holding part 220. The fitting portion 264 may be replaceable. There may be provided a several types of fitting portions 264. In the present embodiment, the fitting portion 264 and the holding part 220 are coupled when manufacturing the second handpiece 200. Further, the fitting portion 295 and the pressing part 235 are also coupled.

### <Method of Using Ophthalmic surgical system>

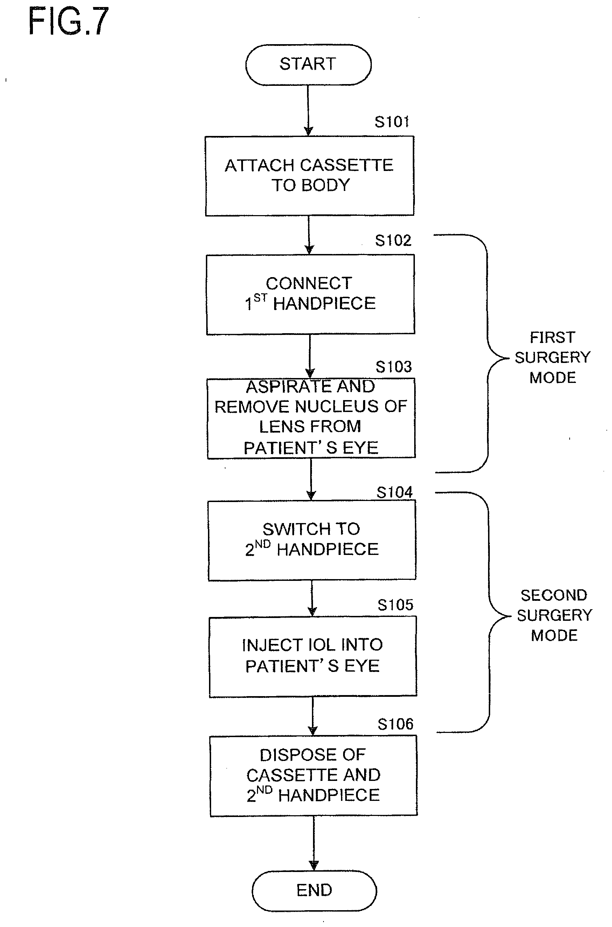

A method of using the ophthalmic surgical system 1 of the present embodiment is explained with reference to FIG. 7. The following explanation exemplifies a cataract surgery using the ophthalmic surgical system 1 of the present embodiment as one example, and specifically, the surgery is carried out in a manner that the nucleus of lens is aspirated and removed from the patient's eye E in a first surgery mode, and then the IOL 400 is injected into a lens capsule of the patient's eye E in a second surgery mode.

The ophthalmic surgical system 1 of the present embodiment includes a plurality of surgery modes (operation modes). Specifically, the ophthalmic surgical system 1 of the present embodiment includes the first surgery mode and the second surgery mode. In the present embodiment, the first surgery mode is set for surgery using the first handpiece 300, and the second surgery mode is set for surgery using the second handpiece 200. The first surgery mode and the second surgery mode of the present embodiment are different in their drive control of the aspiration pump 40, drive control of the drive mechanism 19 (for moving the supply bottle 13 upward and downward), and others. The first surgery mode and the second surgery mode of the present embodiment are different in their setting of the aspiration pressure or setting of the aspiration flow rate. In the present embodiment, switching of the surgery modes can be made by operating the touch screen 11a. In a step S103, which is explained below, the first surgery mode is selected, and the second surgery mode is selected in a step S105. As alternative, the surgery mode may be automatically set by detecting a type of the handpiece by the ophthalmic surgical system 1.

Firstly, in a step S101, a user (an operator, an assistant, or the like) attaches the cassette 20 to the cataract surgical apparatus body 10 (see FIG. 1). In a step S102, the user connects the first handpiece 300 to the cataract surgical apparatus body 10 which has been attached with the cassette 20. To be specific, each connection of the end portion 6a with the terminal 301, the end portion 7a with the terminal 302, and the first handpiece 300 with the cataract surgical apparatus body 10 via the cable 310 is made. In the step S102, connection of the supply bottle 13 which has been poured with the supply liquid to the cassette 20 is also made. Subsequently, in the step S103, the user operates the first handpiece 300 to aspirate and remove the nucleus of lens from the patient's eye E. Specifically, the front end portion of the first handpiece 300 is injected into the patient's eye E, and then the nucleus of lens is destroyed by ultrasonic wave vibration of a cracking tip which is provided on a tip end of the first handpiece 300. More specifically, the supply valve 73 is opened to inject the supply liquid into the patient's eye E. Further, the aspiration pump 40 is driven to aspirate the liquid and others (such as the supply liquid and the nucleus of lens) from the patient's eye E. These operations are carried out by operating the foot pedal 18, the touch screen 11 a, and others.

In the first surgery mode of the present embodiment, both the aspiration pressure and the aspiration flow rate can be determined. For example, the aspiration pressure can be set in a range of 0 to 600 mmHg. Further, the aspiration flow rate can be set in a range of 0 to 60 ml/min. Setting of the aspiration pressure regulates rotation of the rotation part 40a such that the pressure does not exceed the set aspiration pressure. Setting of the aspiration flow rate leads to rotation of the rotation part 40a such that the set aspiration flow rate is maintained. In the present embodiment, the aspiration pressure (the aspiration flow rate) is changeable according to a pressed amount of the foot pedal 18. For example, even when the aspiration flow rate is set as 30 ml/min, the liquid or the like can be aspirated at the aspiration flow rate (in the range of 0 to 30 ml/min) corresponding to the pressed amount of the foot pedal 18. The liquid or the like aspirated from the patient's eye E is gathered in the bag 20b. The controller 80 of the present embodiment is configured to detect the pressure (the aspiration pressure) in the aspiration passage 9 during aspiration based on the output signal from the pressure sensor 70. The controller 80 is, for example, configured to control the rotation speed of the rotation part 40a based on the detected aspiration pressure. The aspiration flow rate can be detected from a rotation amount of the rotation part 40a. The controller 80 is configured to control the aspiration flow rate not to exceed the set value in a case that the detected aspiration pressure is going to exceed a predetermined threshold value, for example. As one example of this control, the pinch valve 74 may be opened when an inflow hole provided in a front end portion of the first handpiece 300 is blocked.

When aspiration and removal of the nucleus of lens are completed, the process proceeds to a step S104. In the step S104, the user switches (exchanges) a handpiece to be connected to the cataract surgical apparatus body 10 from the first handpiece 300 to the second handpiece 200. Specifically, the end portion 6a is connected to the second terminal 256, and the end portion 7a is connected to the first terminal 254 (see FIG. 2). When using the second handpiece 200, the cable 310 is unnecessary. In the present embodiment, replacement of the supply bottle 13 and the cassette 20 is not necessary at the time of shifting the surgery mode from the first surgery mode to the second surgery mode. Namely, the ophthalmic surgical system 1 of the present embodiment can share at least the cassette 20 in the first surgery mode and the second surgery mode (in other words, the cassette 20 can be used repeatedly). Accordingly, after the nucleus of lens is aspirated and removed from the patient's eye E, for example, injection of the IOL 400 can be promptly performed.

Subsequently, in the step S105, the user operates the second handpiece 200 to inject the IOL 400 into the lens capsule of the patient's eye E. To be specific, the front end portion of the second handpiece 200 (the front end portion of the injection part 218) is inserted in the patient's eye E, and then the IOL 400 is injected from the front end portion of the second handpiece 200. The operator places the IOL 400 injected into the eye in the lens capsule which has been removed with the nucleus of lens. In the second surgery mode of the present embodiment, the aspiration flow rate and the aspiration pressure are automatically determined. The ophthalmic surgical system 1 of the present embodiment is configured to perform injection of the IOL 400 by operating the foot pedal 18. When the aspiration flow rate is automatically set as 35 ml/min in the second surgery mode, for example, the aspiration flow rate may be changed in a range of 0 to 35 ml/min according to the pressed amount of the foot pedal 18. In other words, the push-out speed of the IOL 400 (i.e., the moving speed of the plunger 236) may be adjusted according to the pressed amount of the foot pedal 18.

When injection of the IOL 400 is completed, the process proceeds to a step S106. In the step S106, the user pulls out the second handpiece 200 from the patient's eye E, and then disposes of the cassette 20 and the second handpiece 200. Further, for example, cleaning and sterilization of the first handpiece 300 is performed. It is preferable to perform cleaning of the first handpiece 300 swiftly. In the present embodiment, an autoclave (a highpressure sterilizer) is used for sterilizing the first handpiece 300. Herein, the second handpiece 200 of the present embodiment is a disposable type, and thus cleaning and sterilization of the second handpiece 200 at the site of use is unnecessary. The casing body 250 may be shared or repeatedly used, and the cassette 20 and the injector part 210 may be disposed of. The subsequent surgery for another patient is to be then carried out with a new second handpiece 200 which has been sterilized in the process of manufacturing. Further, as mentioned above, the ophthalmic surgical system 1 of the present embodiment is configured such that aspiration and removal of the nucleus of lens and injection of the IOL 400 are performed only by replacing the handpieces (in other words, only by changing connecting directions of the end portion 6a and the end portion 7a). Accordingly, the IOL 400 can be injected with no need of additional equipment which is to be placed around a patient. Further, the ophthalmic surgical system 1 of the present embodiment is configured to move the IOL 400 with a motive power other than the force of the operator's hand. Accordingly, even an operator who is unfamiliar with operation of an intraocular lens injection instrument can easily inject the IOL 400, for example.

### <Detailed explanation of Second surgery mode (Injection of Intraocular lens)>

Detailed explanation of the second surgery mode (injection of IOL) is given with reference to FIGs. 6 and 8 to 11. The following explanation is made on condition that the aspiration amount of the aspiration pump 40 is set at a fixed value. As explained with FIG. 7, replacement of the first handpiece 300 to the second handpiece 200 and switching of the the first surgery mode to the second surgery mode are carried out from the step S104. The controller 80 of the present embodiment detects switching of the surgery mode to the second surgery mode and then initializes the ophthalmic surgical system 1 (for the second surgery mode). This initialization control for the second surgery mode includes, for example, adjustment of the supply bottle 13 in an upward and downward moving position, adjustment of a moving position of the piston part 290, closing of the pinch valve 74, and others.

### <Initialization control>

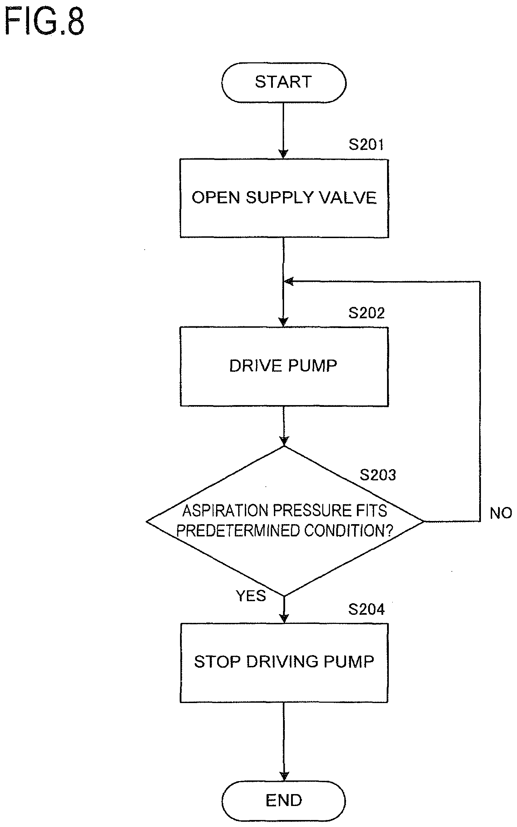

FIG. 8 is a flow chart for explaining the initialization control for the second surgery mode which is carried out by the controller 80 of the present embodiment. By the control shown in the flow chart of FIG. 8, the supply passage 8, the pressure chamber RM, and the aspiration passage 9 are filled with the supply liquid. Further, the piston part 290 is moved backward until the moving wall 291 is brought into contact with the rear wall 284. This retreat is made in the proximal direction of the moving axis L2, which means a direction away from the restriction member 288. The control process through a step S201 to a step S204 which will be explained below is to be performed at a valve-closing state of the pinch valve 74.

In the step S201, the controller 80 performs the control to open the supply valve 73. When the supply valve 73 is opened, the pressure inside the pressure chamber RM increases, so that the piston part 290 is moved toward the proximal end. When the moving wall 291 and the rear wall 284 are brought into contact, the retreat movement of the piston part 290 is stopped. The controller 80 of the present embodiment proceeds to the control in a step S202 after a waiting time since opening of the supply valve 73 has elapsed. In the present embodiment, the maximum time duration for the moving wall 291 moving from the restriction member 288 to the rear wall 284 has been obtained by simulations or experiments, and this maximum time duration is stored in the memory 101 as the above-mentioned waiting time. Herein, the casing body 250 may be formed with a slider protrusion coupled with the piston part 290, and the piston part 290 may be moved backward by sliding the slider protrusion with the force of the user's hand (to bring the moving wall 291 and the rear wall 284 to be brought in contact). Alternatively, the casing body 250 may be provided with a lock mechanism to restrict movement of the piston part 290 toward the front end of the moving axis L2. In this example, the moving wall 291 and the rear wall 284 may be locked in a contact state when manufacturing the second handpiece 200, and the contact state may be unlocked at the site of use.

Referring to FIG. 8, in the step S202, the controller 80 starts driving the aspiration pump 40. To be specific, the controller 80 drives the aspiration pump 40 to generate the negative pressure in the aspiration passage 9. To be more specific, the controller 80 performs the control to rotate the rotation part 40a at a certain rotation speed (for example, the rotation speed for the aspiration flow rate of 35 ml/min) in a direction indicated with a reference sign U (see FIG. 6 (a)). The control process from the step S202 to the step S204 is preferably performed in a state in which the first terminal 254 and the second terminal 256 face upward and the injection part 218 is inclined slightly upward. Thus, it is easy to fill the pressure chamber RM with the supply liquid. In other words, the gas (air) inside the pressure chamber RM is easily drawn out by the aspiration pump 40. Herein, whether or not the gas inside the pressure chamber RM is preferably aspirated may be indicated on the monitor 11 based on the output signal from the pressure sensor 70.

Subsequently, the controller 80 determines whether or not the aspiration pressure fits the predetermined condition in a step S203. The controller 80 proceeds to a step S204 when the aspiration pressure fits the predetermined condition, and when the pressure does not fit the condition, the process returns to the step S202. The controller 80 in the step S204 halts driving (aspiration) of the aspiration pump 40. In other words, the controller 80 of the present embodiment controls the rotation part 40a to keep rotating until the aspiration pressure fits the predetermined condition. During the process from the step S202 to the step S204, the controller 80 controls the rotation part 40a to rotate at a certain rotation speed. The above-mentioned step S203 is explained in detail. The controller 80 of the present embodiment detects the pressure value (the aspiration pressure) in the aspiration passage 9 by the output signal from the pressure sensor 70. The controller 80 repeats detection of the pressure value in the aspiration passage 9 to obtain a pattern of chronological changes in the aspiration pressure. In the step S203, the controller 80 compares a reference pattern which has been stored in advance in the memory 101 with the chronological-change pattern which is obtained by the output signal from the pressure sensor 70. When the reference pattern and the chronological-change pattern correspond to each other, the controller 80 determines that the aspiration pressure fits the predetermined condition. The reference pattern of the present embodiment has been stored in the memory 101 based on the results of simulations, experiments, and others in manufacturing the ophthalmic surgical system 1. Alternatively, a flow rate value of bringing the air out of the aspiration passage 9 may be measured and stored in advance in the memory 101 so that the aspiration pump 40 is rotated according to this flow rate information.

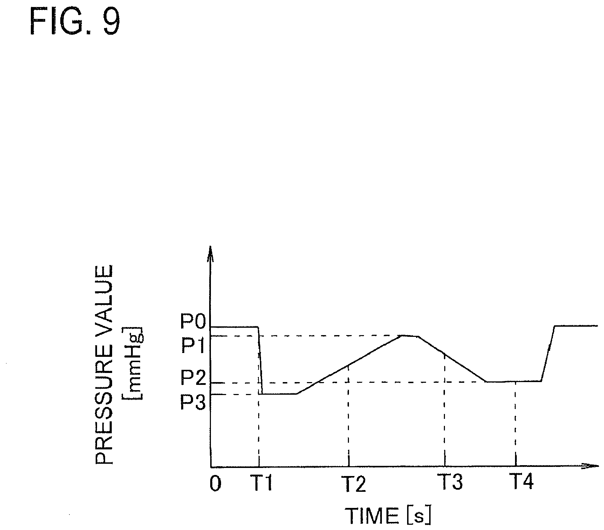

Changes in the aspiration pressure in the aspiration passage 9 during the control process from the step S201 to the step S204 are explained with reference to FIG. 9. FIG. 9 is a graph showing the output signal from the pressure sensor 70 (i.e., the aspiration pressure in the aspiration passage 9). When the rotation part 40a begins to rotate at a time T1, the pressure in the aspiration passage 9 gradually decreases by the negative pressure generated by the aspiration pump 40. The second surgery mode of the present embodiment is preformed after the first surgery mode, and thus the supply passage 8 and the aspiration passage 9 are filled with the liquid (such as the supply liquid). On the other hand, the second handpiece 200 is as good as new, and thus the pressure chamber RM is filled with gas (air) (see FIG. 6(a)). When the liquid which has been filled in advance in the aspiration passage 9 is aspirated by the aspiration pump 40, the pressure value in the aspiration passage 9 decreases from a pressure P0 to a pressure P3. In association with this change in the pressure value in the aspiration passage 9, the gas in the pressure chamber RM moves to the aspiration passage 9. Simultaneously, the liquid (the supply liquid) is supplied into the pressure chamber RM from the supply member.

As mentioned above, the steps S201 to S204 of the present embodiment are performed in a state in which the first terminal 254 faces upward and the injection part 218 is inclined upward. Accordingly, the gas in the pressure chamber RM is let flown out through the first terminal 254 and the liquid flowing from the second terminal 256 are gathered in a bottom of the pressure chamber RM. As a result, during aspiration by the aspiration pump 40, the fluid in the pressure chamber RM is gradually replaced from the gas (the air having been existed in advance in the pressure chamber RM) to the liquid (the supply liquid supplied from the supply passage 8). The air having been existed in advance in the pressure chamber RM moves in the aspiration passage 9, and accordingly, the aspiration resistance in the aspiration passage 9 decreases, so that the pressure value in the aspiration passage 9 increases from the pressure P3 to a pressure P1 (see a point indicated with a time T2 in FIG. 9). Namely, the controller 80 can detect the state in which the gas is aspirated from the pressure chamber RM by the changes in the output signal from the pressure sensor 70.

When the aspiration by the aspiration pump 40 is continuously made, the pressure chamber RM gets filled with the liquid (the supply liquid) and the liquid is then aspirated from the pressure chamber RM. Thus, the fluid in the aspiration passage 9 is shifted from the gas (the air having been existed in the pressure chamber RM) to the liquid (the supply liquid supplied from the supply passage 8). This shift of the fluid brings increase in the aspiration resistance in the aspiration passage 9, thereby decreasing the pressure value in the aspiration passage 9 from the pressure P1 to a pressure P2 (see a point indicated with a time T3 in FIG. 9). The controller 80 can thus detect a state in which the liquid is being aspirated from the pressure chamber RM by the changes in the output signal from the pressure sensor 70. When all the components of the supply passage 8, the pressure chamber RM, and the aspiration passage 9 are filled with the fluid (the supply liquid), the pressure value in the aspiration passage 9 becomes stable at the pressure P2 (see a point indicated with a time T4). Specifically, the controller 80 can detect a balanced state of the aspiration pressure in the aspiration passage 9 by the changes in the output signal from the pressure sensor 70. The controller 80 of the present embodiment is configured to compare the chronological change pattern of the pressure value in the aspiration passage 9 shown in FIG. 9 with the reference pattern which has been stored in advance in the memory 101, and thus the controller 80 detects whether or not the passage extending from the supply bottle 13 to the aspiration pump 40 is filled with the supply liquid.

By the above-mentioned control through the steps S201 to S204, the supply passage 8, the pressure chamber RM, and the aspiration passage 9 are filled with the supply liquid. As a result of this, for example, the movement (the advance movement) of the piston part 290 in injecting the IOL 400 can be performed accurately. By the control through the steps S201 to S204, the piston part 290 is moved backward until the moving wall 291 comes to contact with the rear wall 284. Thus, a piston position is made uniform, thereby restraining deformation variations of the IOL 400 in injecting the IOL 400, for example. The pressure chamber RM may be filled with the fluid when manufacturing the casing body 250. In this case, the connection of the second handpiece 200 and the supply member is not necessary. In other words, when the pressure chamber RM has been filled in advance with the liquid, for example, the ophthalmic surgical system 1 may not be provided with the supply member. Namely, the second handpiece 200 only has to be provided with an opening (as one example, the second terminal 256) for filling the pressure chamber RM with the liquid.

### <Injection of Intraocular lens>

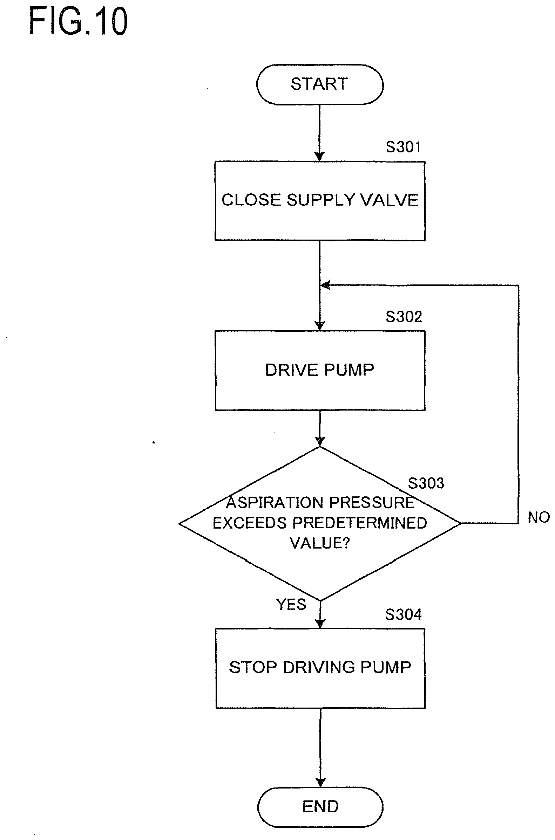

Injection control of the IOL 400 performed by the controller 80 is now explained with reference to FIGs. 6 and 10. Injection of the IOL 400 gets started by pressing the foot pedal 18 after the initialization control (the control through the steps S201 to S204) has been carried out. Control through steps S301 to S304 which will be explained below is performed at the valve-closing state of the pinch valve 74. Firstly, in the step S301, the controller 80 performs the control to close the supply valve 73. The state of the ophthalmic surgical system 1 in the step S301 corresponds to FIG. 6(b). Subsequently, in the step S302, the controller 80 starts driving the aspiration pump 40. Specifically, the controller 80 drives the aspiration pump 40 to generate the aspiration pressure (the negative pressure) in the aspiration passage 9. To be more specific, the controller 80 controls the rotation part 40a to rotate at a certain rotation speed (for example, a rotation speed for the aspiration flow rate of 35 ml/min) in the direction indicated with the reference sign U (see FIG. 6(a)). In the step S303, subsequently, the controller 80 determines whether or not the aspiration pressure reaches the predetermined value or more. Specifically, the controller 80 determines whether or not the aspiration pressure in the aspiration passage 9 exceeds the predetermined value which is stored in the memory 101 by the output signal from the pressure sensor 70. The predetermined value of the present embodiment has been obtained by the results from the simulations, experiments, and others and stored in the memory 101 when manufacturing the ophthalmic surgical system 1. When the aspiration pressure exceeds the predetermined value, the process proceeds to the step S304. When the aspiration pressure is equal to or less than the predetermined value, the process returns to the step S302. The controller 80 in the step S304 halts driving (aspiration) of the aspiration pump 40. Namely, the controller 80 of the present embodiment is configured to continue aspiration by the aspiration pump 40 until the aspiration pressure exceeds the predetermined value.

The above-mentioned step S303 is explained in detail. FIG. 11 is a graph showing changes in the output signal from the pressure sensor 70 when the controller 80 carries out the control through the steps S301 to S304. When driving (aspiration) of the aspiration pump 40 is started at a time T5, the pressure value in the aspiration passage 9 gradually decreases from the pressure P0 to a pressure P5. The pressure inside the pressure chamber RM also decreases in association with the pressure change in the aspiration passage 9. When the pressure in the pressure chamber RM decreases, the piston part 290 is moved toward the front end. According to the movement of the piston part 290, the plunger 236 coupled with the piston part 290 is also moved toward the front end of the moving axis L1 (the moving axis L2). The plunger 236 is brought into contact with the support part of the IOL 400 which is placed on the setting part 219, and then the entire IOL 400 is moved toward the front end of the moving axis L1. The IOL 400 advances in the hollow part 217 having a uniform sectional area, and accordingly, the pressure value in the aspiration passage 9 is almost in a balanced state at the pressure P5 (see a point indicated with a time T6 in FIG. 11).

When the aspiration by the aspiration pump 40 continues and the IOL 400 enters in the injection part 218, the IOL 400 is deformed to be in a smaller shape (see FIG. 6(c)). A frictional force between an inner wall of the injection part 218 and the IOL 400 is gradually increased as the IOL 400 is deformed to be small. The aspiration pump 40 of the present embodiment aspirates the liquid, and the rotation part 40a rotates at a certain rotation speed. Accordingly, the more the above-mentioned frictional force is increased, the lower the pressure value in the aspiration passage 9 becomes (the negative pressure increases). Specifically, the pressure value in the aspiration passage 9 decreases from the pressure P5 to a pressure P6 (see a point indicated with a time T7 in FIG. 11). Thus, in the present embodiment, increase in the aspiration pressure maintains the advancing speed of the plunger 236. Further, the controller 80 of the present embodiment is configured to detect a load applied to the plunger 236 (the load applied in a front and rear direction in FIG. 3) by detecting the aspiration pressure. The advancing speed of the plunger 236 may be changed according to the changes in the pressure value of the aspiration passage 9. Specifically, the advancing speed of the plunger 236 may be changed by varying the rotation speed of the rotation part 40a (i.e., the aspiration flow rate). Alternatively, the advancing speed of the plunger 236 may be changed according to the pressed amount of the foot pedal 18. In other words, the rotation speed of the rotation part 40a may be changed according to the pressed amount of the foot pedal 18. Further alternatively, the changes in the advancing speed of the plunger 236 (changes in the rotation speed of the rotation part 40a) may be programmed in advance. Such a program may be stored in the memory 101. Thus, only by pressing the foot pedal 18, the advancing speed of the plunger 236 can be automatically changed.

When the rotation part 40a is rotated to further move the plunger 236 forward, the IOL 400 gets injected (come out) from the front end of the injection part 218. As the IOL 400 being injected from the injection part 218, the frictional force between the inner wall of the injection part 218 and the IOL 400 gradually decreases. Accordingly, the pressure value in the aspiration passage 9 increases (the negative pressure is relaxed). To be specific, the pressure value in the aspiration passage 9 increases from the pressure P6 to the pressure P5 (see a point indicated with a time T8 in FIG. 11). Further rotation of the rotation part 40a and further movement of the plunger 236 lead to the entire injection of the IOL 400 injected out of the injection part 218. When the IOL 400 is completely injected, the load applied on the plunger 236 decreases all at once. To be specific, the pressure value in the aspiration passage 9 is kept in an almost balanced state at the pressure P5 (see a time T9 in FIG. 11). When the rotation part 40a is further rotated and the plunger 236 has got out of the front end of the injection part 218, the moving wall 291 and the restriction member 288 are brought into contact (see FIG. 6 (d)). As a result of this, the pressure value in the aspiration passage 9 largely decreases (the negative pressure increases at once). The pressure value in the aspiration passage 9 is largely changed from the pressure P5 to a pressure P8 (see a time T10 in FIG. 11). When the controller 80 of the present embodiment detects the pressure P8, the controller 80 halts driving (aspiration) of the aspiration pump 40 (the steps S303 and S304). When driving of the aspiration pump 40 is stopped, the pressure value in the aspiration passage 9 becomes stable at the pressure P8. Injection of the IOL 400 by use of the second handpiece 200 is thus carried out in the present embodiment.

The ophthalmic surgical system 1 of the present embodiment is configured to detect the moving position of the plunger 236 by the output signal from the pressure sensor 70 provided on the aspiration passage 9. In other words, the ophthalmic surgical system 1 of the present embodiment is configured to use the output signal from the pressure sensor 70 provided on the aspiration passage 9 to detect whether or not the IOL 400 has beeb injected from the second handpiece 200. The ophthalmic surgical system 1 of the present embodiment is further configured to detect the load (the push-out load) applied to the plunger 236 by use of the output signal from the pressure sensor 70 provided on the aspiration passage 9. Thus, for example, inappropriate push-out of the IOL 400 can be easily detected. Herein, the pinch valve 74 may be opened after halting driving of the aspiration pump 40 (i.e., after the step S304). Opening the pinch valve 74 facilitates increase in the aspiration pressure (relaxing the negative pressure) in the aspiration passage 9. More specifically, by opening the pinch valve 74, the pressure value in the aspiration passage 9 increases from the pressure P8 to the pressure P9 (the supply pressure) (see a time T11 in FIG. 11). Increase in the pressure value in the aspiration passage 9 leads to movement of the plunger 236 (the piston part 290) toward the proximal end of the moving axis L1 (the moving axis L2). By this control of the pressure value, it is possible to bring the plunger 236, which has come out of the front end of the injection part 218, back to the inside of the injection part 218, and then pull out the injection part 218 from the patient's eye E, for example. Accordingly, for example, it is possible to prevent the plunger 236 from being contacted with the patient's eye E. Further, opening the pinch valve 74 causes relaxation in the negative pressure in the aspiration passage 9 and in the pressure chamber RM, thereby facilitating disconnection of the second handpiece 200 from the tube (the tube 6 or the tube 7).

In the present embodiment, by detecting the pressure in the aspiration passage 9, connection of the moving wall 291 with the restriction member 288 is detected. As alternative to this, an abnormal advancing of the plunger 236 may be detected by detecting the pressure in the aspiration passage 9. For example, when the pressure value in the aspiration passage 9 exceeds the pressure P7 (see FIG. 11), the monitor 11 may be displayed with an alarm message. Further alternatively, the advancing position of the plunger 236 may be detected from the rotation speed of the rotation part 40a and the pressure value in the aspiration passage 9. Moreover, as mentioned above, opening of the pinch valve 74 brings retreat of the plunger 236 in the present embodiment. Namely, the ophthalmic surgical system 1 of the present embodiment includes a retreat member to bring backward the plunger 236, which has been moved forward, by the aspiration member. When the pressure value in the aspiration passage 9 exceeds the pressure P7 (see FIG. 11) during pushing out the IOL 400, for example, the plunger 236 may be controlled to move backward. Alternatively, the pinch valve 74 may be opened just one moment to bring the plunger 236 slightly backward. The retreat method for the plunger 236 is not limited to opening of the pinch valve 74. For instance, the rotation part 40a may be rotated reversely (in a reverse direction from the direction indicated with the reference sign U in FIG. 6 (a)).

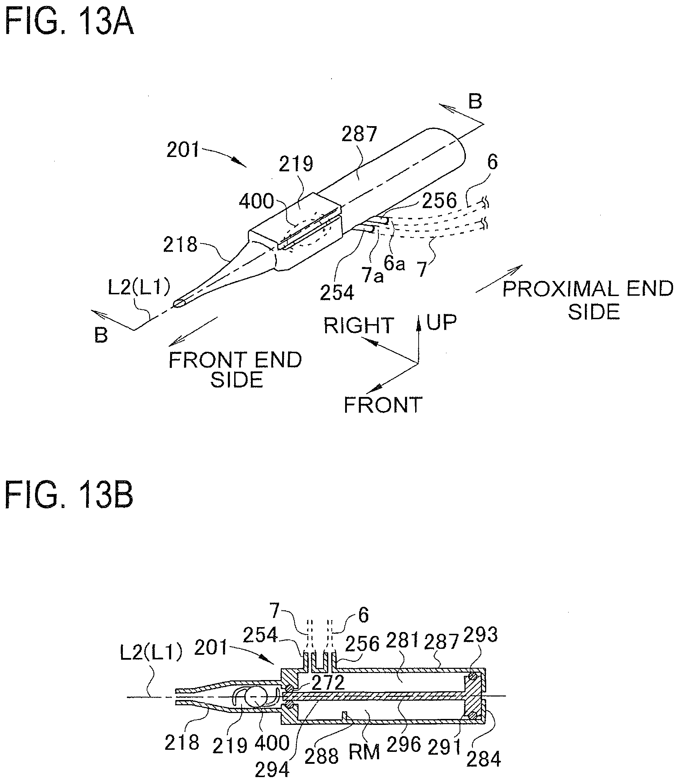

As shown in FIGs. 13A and 13B, for example, a second handpiece 201 (an intraocular lens injection instrument in a modified example) is configured such that the above-mentioned injector part 210 and the casing body 250 are integrally formed. FIG. 13A is a schematic perspective view of the second handpiece 201 (the intraocular lens injection instrument) when it is seen from an obliquely upper direction, and FIG. 13B is a sectional view taken along a line B-B in FIG. 13A. The second handpiece 201 includes a casing body 287 and a piston part 296. The second handpiece 201 further includes a pressure chamber RM. The casing body 287 includes a first terminal 254, an injection part 218, a hollow part 281, and others. The piston part 296 includes a moving wall 291, a movable member 294 (a plunger), and others. In the figures, the same components allotted with the same reference signs with FIGs. 3 to 5 are omitted with their explanation. The second handpiece 201 is configured such that the IOL 400 is directly pushed by the piston part 296. Specifically, the movable member 294 of another embodiment is configured to directly push out the IOL 400. Herein, the IOL 400 may be loaded in advance as similar to the above-mentioned second handpiece 200. The second handpiece 201 is configured more simply than the second handpiece 200. For the second handpiece 201, too, the second terminal 256 may not be provided. Namely, only an aspiration member may be connected to the second handpiece 201.

In the present embodiment, the first handpiece 300 and the second handpiece 200 share the aspiration member (the cataract surgical apparatus body 10). Alternatively, the configuration may be a combination of the second handpiece 200 and another aspiration member. The aspiration member to be connected to the second handpiece 200 is not limited to the one used for aspiration and removal of the nucleus of lens of the patient's eye E. Further, in the present embodiment, the IOL 400 is injected in a state in which the aspiration passage 9 and the pressure chamber RM are filled with the liquid. Alternatively, the aspiration passage 9 and the pressure chamber RM may be filled with gas as the fluid. Further alternatively, a venturi pump may be adapted for the aspiration pump 40.

### <Summary>

The second handpiece 200 (the intraocular lens injection instrument) of the present embodiment is provided with the casing body 250 including the pressure chamber RM which is variable in its volume by the moving wall 291 provided movable in the casing, the first terminal 254 communicated with the pressure chamber RM to connect the pressure chamber RM with the aspiration pump 40 via the tube 7, and the movable member 294 having one end connected with the moving wall 291 and the other end for directly or indirectly pushing out the IOL 400. The second handpiece 200 is configured to convert the aspiration force of the aspiration pump 40 into a linear movement of the movable member 294 by the movement of the moving wall 291 moved by the negative pressure in the pressure chamber RM which is generated by the aspiration force of the aspiration pump 40. Specifically, the second handpiece 200 of the present embodiment includes a conversion mechanism to convert the aspiration force of the aspiration pump 40 into the linear movement of the movable member 294 by the movement of the moving wall 291 moved by the negative pressure in the pressure chamber RM, the pressure being generated by the aspiration force of the aspiration pump 40. Thus, the IOL 400 can be easily pushed out without a force of one's hand, for example.

Further, the second handpiece 200 of the present embodiment is provided with the second terminal 256 for flowing the liquid into the pressure chamber RM. Thus, the IOL 400 can be accurately pushed by the aspiration force of aspirating the liquid, for example. Further, the first terminal 254 of the second handpiece 200 of the present embodiment is communicated with the pressure chamber RM at a position far front than a moving limit position of the moving wall 291 which is movable in the pressure chamber RM. Accordingly, for example, it is possible to prevent such a situation that a through hole communicating the first terminal 254 with the pressure chamber RM is blocked by the moving wall 291 during movement of the movable member 294, and as a result of this, the aspiration force of the aspiration pump 40 fails to be transmitted to the pressure chamber RM.

The inner wall 286 of the pressure chamber RM of the second handpiece 200 according to the present embodiment is provided with the restriction member 288 to define the moving limit in the linear movement of the moving wall 291. It is thus possible to prevent unnecessary advance (forward movement) of the movable member 294 after injection of the IOL 400. Further, the movable member 294 and the casing body 250 of the second handpiece 200 are made of resin in the present embodiment. The second handpiece 200 is therefore easy to be disposed of, and further easy to be manufactured inexpensively.

The ophthalmic surgical system 1 of the present embodiment is provided with the cataract surgical apparatus body 10 including the second handpiece 200 and the aspiration pump 40, the foot pedal 18 to drive the aspiration pump 40, and the tube 7 to connect the aspiration pump 40 with the first terminal 254 provided in the second handpiece 200. This configuration makes it easy to inject the IOL 400 by use of the driving force of the aspiration pump 40, for example. Further, the ophthalmic surgical system 1 of the present embodiment is provided with the pressure sensor 70 provided in the aspiration passage 9 of the aspiration pump 40 to detect the pressure in the aspiration passage 9 and the controller 80 (a drive control member) to control driving of the aspiration pump 40 by the output signal from the pressure sensor 70. This configuration enables preferable movement of the movable member 294, for example.

The controller 80 (the drive control member) of the ophthalmic surgical system 1 of the present embodiment is configured to attenuate the aspiration force generated by the aspiration pump 40 in a case that the pressure in the aspiration passage 9 exceeds the predetermined value. This configuration enables to prevent the movable member 294 from advancing (moving forward) more than necessary, and further preferably prevent the IOL 400 from getting damaged and others, for example.

The ophthalmic surgical system 1 of the present embodiment adapts a peristaltic aspiration pump as the aspiration pump 40. Thus, the fluid in the pressure chamber RM can be aspirated at a certain flow rate, for example. As a result, the movable member 294 can be accurately moved. Further, the ophthalmic surgical system 1 of the present embodiment is provided with the first handpiece 300 to crack and aspirate the nucleus of lens, and the first handpiece 300 is configured to be connected to the aspiration pump 40. Accordingly, both aspiration of the nucleus of lens and injection of the IOL 400 can be performed by the single cataract surgical apparatus body 10, for example.

A cataract surgical method using the cataract surgical apparatus body 10 of the present embodiment includes a first step of connecting the first handpiece 300 to the cataract surgical apparatus body 10, a second step of cracking and aspirating the nucleus of lens of the patient's eye E by the first handpiece 300 and the aspiration pump 40, a third step of replacing the first handpiece 300 with the second handpiece 200 as a handpiece to be connected to the cataract surgical apparatus body 10, and a forth step of injecting the IOL 400 into the patient's eye E by the second handpiece 200 and the aspiration pump 40. According to this method, cracking and aspiration of the nucleus of lens of the patient's eye E and injection of the IOL 400 into the patient's eye E are carried out. The cataract surgical method of the present embodiment using the cataract surgical apparatus body 10 is performed by sharing the cassette 20 in the second step and in the forth step.

The above disclosed embodiments are only illustration in all the aspects and not limitative. The scope of the present disclosure is defined not by the above explanation but by the appended claims, and the scope of the disclosure is intended to include the appended claims and any modifications equivalent to and within that scope.

## Claims

1. An intraocular lens injection instrument (200) comprising:
a casing body (250) including a pressure chamber (RM) which is variable in its volume by movement of a moving wall (291) provided movably in a housing;
a first terminal (254) configured to communicate with the pressure chamber (RM) for connecting an aspiration pump (40) via a tube (7); and
a movable member (294) having one end connected with the moving wall (291) and the other end configured to directly or indirectly push an intraocular lens (400), wherein
the moving wall (291) is movable by a negative pressure to be generated by an aspiration force of the aspiration pump (40), and the moving wall (291) is configured to be moved to convert the aspiration force of the aspiration pump (40) into linear movement of the movable member (294).

2. The intraocular lens injection instrument (200) according to claim 1 further comprising a second terminal (256) to make liquid flow into the pressure chamber (RM).

3. The intraocular lens injection instrument (200) according to claim 1 or 2, wherein the first terminal (254) communicates with a part of the pressure chamber (RM), the part being located far front than a moving limit position of the moving wall (291) which is movable in the pressure chamber (RM).

4. The intraocular lens injection instrument (200) according to any one of claims 1 to 3, wherein an inner wall (286) of the pressure chamber (RM) is provided with a restriction member (288) for defining a moving limit of the linear movement of the moving wall (291).

5. The intraocular lens injection instrument (200) according to any one of claims 1 to 4, wherein the movable member (294) and the casing body (250) are made of resin.

6. An ophthalmic surgical system (1) including an intraocular lens injection instrument (200) according to any one of claims 1 to 5 comprising:
a cataract surgical apparatus body (10) provided with the aspiration pump (40);
a foot pedal (18) for driving the aspiration pump (40); and
the tube (7) for connecting the aspiration pump (40) with the first terminal (254) provided in the intraocular lens injection instrument (200).

7. The ophthalmic surgical system (1) according to claim 6 further comprising:
a pressure sensor (70) provided on an aspiration passage (9) of the aspiration pump (40) to detect pressure in the aspiration passage (9); and
a drive control member (80) for controlling drive of the aspiration pump (40) by use of an output signal from the pressure sensor (70).

8. The ophthalmic surgical system (1) according to claim 7, wherein the drive control member (80) is configured to attenuate the aspiration force generated by the aspiration pump (40) when the pressure exceeds a predetermined value.

9. The ophthalmic surgical system (1) according to any one of claims 6 to 8, wherein a peristaltic aspiration pump is used as the aspiration pump (40).

10. The ophthalmic surgical system (1) according to any one of claims 6 to 9, wherein
a handpiece (300) for cracking and aspirating a nucleus of lens is provided, and
the aspiration pump (40) is configured to be connected to the handpiece (300).
